Europäisches Patentamt

European Patent Office

Office européen des brevets

(11) Publication number: **0 462 353 A1**

# EUROPEAN PATENT APPLICATION

(21) Application number: 90830574.1

(22) Date of filing: **11.12.90**

(51) Int. Cl.⁵: **C12Q 1/68**, C12Q 1/70

(30) Priority: **18.06.90 IT 6744590**

(43) Date of publication of application:
**27.12.91 Bulletin 91/52**

(84) Designated Contracting States:
**AT BE CH DE DK ES FR GB GR IT LI LU NL SE**

(71) Applicant: **CONBIOTEC - CONSORZIO PER LE BIOTECNOLOGIE**
**Piazzale Spedali Civili 1**
**I-25123 Brescia(IT)**

(72) Inventor: **Primi, Daniele**
**45 Rue des Volontaires**
**F-75015 Paris(FR)**
Inventor: **Albertini, Alberto**
**Via Marsala 44**
**I-25100 Brescia(IT)**

(74) Representative: **Rambelli, Paolo et al**
**Jacobacci-Casetta & Perani S.p.A. Via Alfieri 17**
**I-10121 Torino(IT)**

(54) **A method for the specific detection of a target nucleotide sequence in a sample of genetic material.**

(57) A method for determining the presence of a target nucleotide sequence in a sample of genetic material comprising placing the sample, in which the genetic material has been made available in single-stranded form, in contact, under hybridisation conditions, with a nucleotide probe substantially complementary to the target sequence, and detecting the formation of a probe/target hybrid, in which the probe/target hybrid is detected with the use of a monoclonal or polyclonal anti-nucleic-acid antibody adapted electively to distinguish double-stranded nucleotide sequences from single-stranded sequences.

EP 0 462 353 A1

The present invention relates to a method for determining the presence of a target nucleotide sequence in a sample of genetic material.

In order to determine the presence or the identity of a specific nucleotide sequence in a genetic material, for example, DNA, it has been proposed to denature the genetic material to bring it to the single-stranded form and to place the denatured genetic material in contact with a marked nucleotide probe which is fixed to a solid, insoluble substrate and has a sequence of bases complementary to the target nucleotide sequence. The probe, in the single-stranded form, is marked with a substance which can provide a measurable signal or can generate a measurable signal. Alternatively, the single-stranded genetic material is fixed to a solid substrate and placed in contact with the marked probe. The single-stranded genetic material and the single-stranded probe are placed in contact under conditions which cause the probe and the target sequence to hybridise, and the formation of the probe/target hybrid is then detected by the direct or indirect detection of the signal associated therewith.

U.S. patent No. 4,358,535 describes a method of the aforesaid type which uses DNA probes marked with radioactive isotopes. However, the disadvantages associated with the use of radioactive markers are known. These disadvantages result not only from the precautions necessary and the risks involved in handling radioactive materials but also from the short life of the radioactive material and the costs involved in the use and handling of radioactive probes.

Methods are also known which provide for the use of probes marked chemically by the incorporation in the probe of a detectable molecule with a specific ligand containing a signal source such as, for example, avidin/biotin and hapten/antibody systems. All these methods, however, require the introduction into the DNA probe of a modifying agent which enables the hybrid to be detected directly or indirectly.

These approaches require numerous steps, both at the analytical stage and at the preparatory stage; on the one hand, numerous incubation and washing sequences are required and, on the other hand, it is necessary accurately to select and standardise all the components of the system for the association and detection of the signal.

The object of the present invention is to provide a method which completely eliminates all the variables associated with the incorporation of the signal and at the same time achieves sensitivity levels comparable with those achieved by radioactive methods.

A subject of the invention is therefore a method for determining the presence of a target nucleotide sequence in a sample of genetic material comprising the placing of the sample, in which the genetic material has been made available in single-stranded form, in contact, under hybridisation conditions, with a nucleotide probe substantially complementary to the target sequence and the detection of the formation of a probe/target hybrid, characterised in that the probe/target hybrid is detected with the use of a monoclonal or polyclonal anti-nucleic-acid antibody adapted electively to distinguish double-stranded nucleotide sequences from single-stranded sequences.

It has been found that it is possible to obtain antibodies, preferably monoclonal antibodies, which can distinguish single-stranded DNA from double-stranded DNA and that these antibodies can be used to detect the formation of specific hybrids. The method proposed thus completely eliminates all the variables associated with the incorporation of the signal since the signal is supplied by the nucleotide sequence itself as a result of the hybridisation reaction. The method is therefore suitable for the specific detection and quantification of nucleic acids, be they DNA of synthetic or biological origin, or RNA of chemical or biological origin.

Within the scope of the present invention, it is particularly preferred to use the anti-DNA monoclonal antibody designated 27-14-D9, produced by the Applicant. This antibody has the property that it recognises double-stranded DNA with an affinity $10^3$ times greater than that which it has for single-stranded DNA. Moreover, the antibody 27-14-D9 has the property, which is very rare for this type of molecule, that it does not fix non-specifically to proteins, such as bovine albumin, which are normally used to saturate the solid phases. This combination of properties thus makes it a particularly suitable reagent for the proposed application. It is intended, however, that anti-DNA monoclonal antibodies useful for carrying out the method of the invention may be produced, for example by the technique described below.

In order to carry out the method of the invention, the oligonucleotide probe specific to the target DNA is preferably immobilised on a solid or insoluble phase. The probe can be immobilised by physical or chemical methods or by interactions such as avidin/biotin, streptavidin/biotin and ligand/anti-ligand interactions.

The probe may be immobilised on a solid or insoluble substrate constituted, for example, by polystyrene, polypropylene, polyvinyl, nylon, borosilicate, cellulose or derivatives thereof, each material being provided in the form of tubes, dishes, macrospheres or microspheres; however the immobilisation may alternatively be carried out on a solid substrate of the denatured genetic material.

The genetic material containing the target DNA is amplified by the known amplification method and the amplification product is denatured thermally or chemically and then cooled so that it assumes and retains the single-stranded conformation and is then incubated with the solid phase to which the oligonucleotide probe is fixed.

During the incubation, if the amplified DNA is complementary to the immobilised probe, the double-stranded hybrids of the probe and the target DNA are immobilised on the solid phase.

The DNA which has not reacted with the probe is removed by washing. Under these conditions, the only double-stranded DNA present in the system is the specific hybrid generated on the solid or insoluble phase.

At this point an anti-DNA antibody which can react electively or selectively with double-stranded DNA is added to the system. The antibody may be marked with a substance which can provide a measurable signal. For this purpose enzymes such as peroxidase, alkaline phosphatase, urease, beta-galactosidase, or fluorescent substances such as fluoroseine, rhodamine, Texas-Red or chemiluminescent substances are preferably used. The antibody which is not fixed is removed and the signal associated with the antibody, which confirms that specific hybridisation has taken place between the probe and the target DNA, is detected.

Alternatively, if the antibody is not marked, a ligand which can react with the antibody itself may be added, the ligand in turn being marked with a substance which can provide a measurable signal of the type indicated above, and the unreacted ligand is then removed.

The analytical scheme outlined above has been applied, in particular, to the detection of the genome of the hepatitis B virus (HBV), amplified in certain regions, and demonstrates the validity of the method in terms of sensitivity, simplicity, specificity and reproducibility.

Example 1

The DNA in the serum of patients infected by hepatitis B was amplified with the use of two primers complementary to two segments, 447 bases apart, of the "core" gene sequence. For the amplification reaction, 1 $\mu$l of serum was mixed in an Eppendorf tube containing 10 $\mu$l of amplification buffer (670 mM Tris HCL pH 8.8, 166 mM $(NH_4)_2SO_4$, 15 M $MgCl_2$, 100 Mm beta-mercaptoethanol, 1 mg/ml bovine albumin) with 200 $\mu$moles of each of the four nucleotides, 50 pmoles of each of the two primers and 5 units of Taq polymerase enzyme. The DNA was amplified by thermal cycles alternated with hybridisation, elongation and denaturing cycles. The amplified DNA was then detected by the method of the invention. An oligonucleotide complementary to a portion within the amplified segment was biotinilated for use as a probe for picking up the amplified DNA. The biotinilation reaction was effected by the mixing of the denatured probe in a 0.1M pH 8.5 borate buffer with 500 moles of biotin-N-hydroxysuccinimide, that is, an excess. The reaction took place at ambient temperature over 16 hours and the biotinilated probe was then separated from the free biotin in a Sephadex G15 column. The material recovered was precipitated with sodium acetate and ethanol and resuspended at the desired concentration in pH 7.5 Tris-EDTA buffer (10 mM Tris 1 mM EDTA). The probe was immobilised on a solid phase (polystyrene dishes) pre-coated with BSA streptavidin. The dishes were incubated for 16 hours at ambient temperature with 100 $\mu$l of TE solution containing 10 ng of the probe. The excess probe was then removed by two washings in TE, one washing in 0.1xSSC, 0.2% SDS and a final washing in TE. The dishes obtained may be used immediately or, alternatively, may be kept for several months at 4°C or in a damp environment.

In order to hybridise the amplified DNA with the probe immobilised on the solid phase, the DNA was denatured for 10 minutes at 100°C and cooled for 10 minutes to 4°C. The DNA was then diluted to the desired concentration in hybridisation solution containing, per one ml, 220 g of Ficoll, 220 g of polyvinylpyrrolidone, 200 $\mu$g BSA, 8.7 mg NaCl and 4.4 mg sodium citrate, pH7.

The solution was then added to the dishes and hybridised at 50°C for 1 hour. The amplified DNA which had not hybridised was then removed by 3 washings in 0.1xSSC and 0.2% SDS and a final washing in TE.

The hybridised DNA was then detected by a monoclonal antibody specific to double-helix DNA. This antibody was obtained by the fusion of spleen cells from a 12-month-old mouse of the strain MRL/1pr. The selection of this particular type of animal was suggested by the fact that mice of this strain develop a genetically transmissible disease characterised by a syndrome similar to that of lupus erythematosus. From a certain age, these animals therefore spontaneously develop large quantities of autoantibodies amongst which those specific to double-helix or single-helix DNA predominate. Many antibodies which could recognise DNA were obtained from the fusion but, of these, only 3 could distinguish double-helix DNA from single-helix DNA. These antibodies were identified by direct binding tests with the use of native or denatured DNA on a solid phase. After cloning, one of these 3 hybridomas, designated 27-14-D9, was found most suitable for detecting the hybridised DNA.

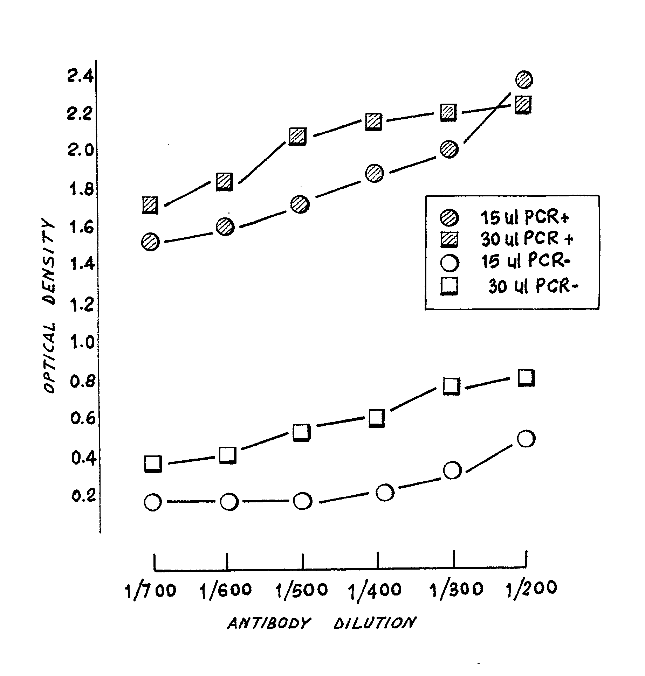

The antibody 27-14-D9 was then incubated for 2 hours at 37°C in the dishes containing the amplified DNA. After 4 washings with 0.05% PBS-Tween, the antibody fixed to the hybridised DNA was detected by means of a second antibody specific to mouse immunoglobulin and marked with peroxidase. This last step may be omitted and the antibody 27-14-D9 marked directly with an enzyme which can develop a colorimetric reaction. After the addition of the enzyme to the specific substrate, the colorimetric reaction is read by a spectrophotometer. Figure 1 shows the results obtained with various quantities of amplification mixture from the serum of patients with hepatitis and from normal serum. The results are expressed as the optical densities read by the spectrophotometer.

Example 2

Graduated quantities of human genome DNA were spotted onto two nitrocellulose filters and fixed by incubation at 80°C in a vacuum oven. The filters were pre-hybridised for 4 hours at 37°C. One of the two filters was then hybridised for 4 hours at 60°C with a 48-base oligonucleotide complementary to a part of the sequence of the C alpha gene of the human T-cell receptor.

The hybridised and non-hybridised filters were then incubated for 2 hours at 37°C with 10 µg of the antibody 27-14-D9, washed and reincubated for 1 hour at 37°C with 100 ng of rabbit antibodies specific to mouse immunoglobulin and marked with peroxidase. After 3 washings the filters were incubated with a precipitating colorimetric substrate and the reaction was stopped after 15 minutes.

The colorimetric reaction was detectable only on the filters hybridised with the oligonucleotide. No signal was detected on the non-hybridised filters. In its current state, the method can detect 5pg of genome DNA.

The scope of the present invention thus extends both to the use of a polyclonal or monoclonal anti-nucleic-acid antibody, which can electively recognise double-stranded nucleotide sequences, for detecting hybridisation between complementary single-stranded nucleic acid sequences, and to diagnostic kits including an anti-nucleic-acid antibody suitable for carrying out hybridisation tests.

**Claims**

1. A method for determining the presence of a target nucleotide sequence in a sample of genetic material comprising placing the sample, in which the genetic material has been made available in single-stranded form, in contact, under hybridisation conditions, with a nucleotide probe substantially complementary to the target sequence, and detecting the formation of a probe/target hybrid, characterised in that the probe/target hybrid is detected with the use of a monoclonal or polyclonal anti-nucleic-acid antibody adapted electively to distinguish double-stranded nucleotide sequences from single-stranded sequences.

2. A method according to Claim 1, in which the anti-nucleic-acid antibody is marked with a substance which can provide a measurable signal.

3. A method according to Claim 1, in which the antibody is not marked and the product of the reaction between the probe/target hybrid and the antibody is detected by the addition of a ligand marked with a substance which can provide a measurable signal and which can react with the antibody, followed by the removal of the unreacted ligand.

4. A method according to any one of Claims 1 to 3, in which the target nucleotide sequence is a viral DNA sequence.

5. A method according to Claim 4, in which the nucleotide sequence is a sequence of the hepatitis B virus.

6. A method according to any one of the preceding claims, in which the antibody is an anti-DNA monoclonal antibody.

7. A method according to any one of Claims 2-6, in which the substance which can provide a measurable signal is selected from the group consisting of radioactive isotopes, enzymes, fluorescent substances and chemiluminescent substances.

8. A method according to any one of the preceding claims, in which the probe is immobilised on a solid substrate.

9. A method according to any one of Claims 1 to 7, in which the genetic material containing the target sequence is denatured and amplified before it is incubated with the probe.

10. The use of a polyclonal or monoclonal anti-nucleic-acid antibody which can electively recognise double-stranded hybridised nucleic-acid sequences in order to detect hybridisation between complementary single-stranded nucleic acid sequences.

11. A diagnostic kit for carrying out hybridisation tests between a target nucleotide sequence and a nucleotide probe, characterised in that it includes an anti-nucleic-acid antibody adapted electively to distinguish double-stranded nucleotide sequences from single-stranded sequences.

European
Patent Office

**EUROPEAN SEARCH
REPORT**

Application Number

**EP 90 83 0574**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 135 159   (MILES, INC.)<br>* Page 1, lines 24-31; page 2, line 17 - page 3, line 10; page 6, line 7 - page 9, line 4; claims 7,8 * | 1,6,10,11 | C 12 Q 1/68<br>C 12 Q 1/70 |
| Y | | 4,5 | |
| Y | EP-A-0 229 701   (CETUS CORP.)<br>* Page 2, line 54 - page 3, line 49; page 10, lines 10-64; page 14, lines 24-65 * | 4,5 | |
| A | ANALYTICAL BIOCHEMISTRY, vol. 181, 1989, pages 96-105, Academic Press, Inc.; F. COUTLEE et al.: "Immunodetection of DNA with biotinylated RNA probes: A study of reactivity of a monoclonal antibody to DNA-RNA hybrids"<br>* Abstract; page 99, column 1, lines 20-40; page 102, column 1, lines 37-40 * | 1,4,6,9 | |
| A | PROC. NATL. ACAD. SCI. USA, vol. 86, January 1989, pages 312-316; S. KANEKO et al.: "Detection of serum hepatitis B virus DNA in patients with chronic hepatitis using the polymerase chain reaction assay"<br>* Abstract; page 312, column 2, line 43 - page 313, column 1, line 19 * | 4,5 | **TECHNICAL FIELDS SEARCHED (Int. Cl.5)**<br><br>C 12 Q |
| T | CLINICAL CHEMISTRY, vol. 37, no. 3, 1991, pages 422-429; G. MANTERO et al.: "DNA enzyme immunoassay: General method for detecting products of polymerase chain reaction"<br>* The whole document * | | |

The present search report has been drawn up for all claims

| Place of search | Date of completion of search | Examiner |
|---|---|---|
| The Hague | 09 August 91 | LUZZATTO E.R.P.G.A. |